# EUROPÄISCHE PATENTSCHRIFT

(11) **EP 3 271 038 B1**
(45) Veröffentlichungstag und Bekanntmachung des Hinweises auf die Patenterteilung: **06.10.2021**
(21) Anmeldenummer: 16710960.2
(22) Anmeldetag: 16.03.2016
(51) Int. Cl.: B01D 3/20, B01D 3/22

(54) **QUERSTROMBODEN FÜR EINE STOFFAUSTAUSCHKOLONNE, STOFFAUSTAUSCHKOLONNE UND VERWENDUNG DER STOFFAUSTAUSCHKOLONNE ZUR TRENNUNG EINES (METH)ACRYLMONOMERE ENTHALTENDEN GEMISCHS**
CROSS-FLOW BASE FOR A MATERIAL EXCHANGE COLUMN, MATERIAL EXCHANGE COLUMN AND USE OF THE MATERIAL EXCHANGE COLUMN FOR SEPARATING A MIXTURE CONTAINING (METH)ACRYLIC MONOMERS
PLATEAU À COURANT TRANSVERSAL D'UNE COLONNE DE TRANSFERT DE MATIÈRE, COLONNE DE TRANSFERT DE MATIÈRE ET UTILISATION DE LA COLONNE DE TRANSFERT DE MATIÈRE AFIN DE SÉPARER UN MÉLANGE CONTENANT DES MONOMÈRES (MÉTH)ACRYLIQUES

(30) Priorität: 18.03.2015 DE 102015204904
(43) Veröffentlichungstag der Anmeldung: 24.01.2018
(73) Patentinhaber: BASF SE, 67056 Ludwigshafen am Rhein (DE)
(72) Erfinder: BLASCHKE, Tim, 70173 Stuttgart (DE); CREMER, Ulrich, 67061 Ludwigshafen (DE); HAMMON, Ulrich, 68163 Mannheim (DE)
(74) Vertreter: BASF IP Association
(86) Internationale Anmeldenummer: PCT/EP2016/055637
(87) Internationale Veröffentlichungsnummer: WO 2016/146657

(56) Entgegenhaltungen:
- DE-A1- 10 257 915
- DE-A1- 10 332 758
- DE-A1-102012 204 436

## Beschreibung

Die Erfindung betrifft einen Querstromboden für eine Stoffaustauschkolonne, in der ein Gas im Gegenstrom zu einer Flüssigkeit geführt wird, wobei der Boden Durchtrittsöffnungen für das Gas und mindestens zwei Ablaufschächte aufweist, wobei die Ablaufschächte über die Oberseite des Querstrombodens hinausragen und unterhalb jedes Ablaufschachtes eine Auffangtasse angeordnet ist. Weiterhin betrifft die Erfindung eine den Querstromboden enthaltende Stoffaustauschkolonne sowie eine Verwendung der Stoffaustauschkolonne.

Derartige Querstromböden finden zum Beispiel Einsatz in der Herstellung von (Meth)acrylmonomeren. Diese umfassen insbesondere Acrylsäure, Methacrylsäure, Acrolein, Methacrolein sowie Ester der Acrylsäure und Ester der Methacrylsäure.

(Meth)acrylmonomere sind wichtige Ausgangsverbindungen zur Herstellung von Polymerisaten, die zum Bespiel als Klebstoffe oder als Wasser-superabsorbierende Materialien in Hygieneartikeln Verwendung finden.

Querstromböden weisen Durchtrittsöffnungen für das Gas auf, die derart dimensioniert sind, dass aufgrund der Gasströmung keine Flüssigkeit durch die Durchtrittsöffnungen vom Boden abläuft. Weiterhin sind Ablaufschächte umfasst, durch die die Flüssigkeit ablaufen kann. In einer Kolonne sind übereinander angeordnete Querstromböden jeweils um 180° zueinander gedreht, so dass durch Ablaufschächte des oberen Bodens ablaufende Flüssigkeit auf den darunter liegenden Boden auf der den Ablaufschächten abgewandten Seite auftrifft, über den Boden zu den Ablaufschächten des Bodens fließt und durch die Ablaufschächte abläuft. Gleichzeitig strömt das aufsteigende Gas durch die quer zur Gasströmung über den Boden fließende Flüssigkeit, wodurch ein intensiver Austausch zwischen Gas und Flüssigkeit erzielt wird.

Eine entsprechende Stoffaustauschkolonne mit Querstromböden, die zur Herstellung von (Meth)acrylmonomeren eingesetzt wird, ist zum Beispiel in WO-A 2013/139590 beschrieben. Hierbei sind unterhalb der Ablaufschächte jeweils Auffangtassen angeordnet, um einen statischen Flüssigkeitsverschluss des Ablaufschachts zu erhalten. Durch den Flüssigkeitsverschluss wird verhindert, dass Gas über den Ablaufschacht aufsteigt. Da sich oberhalb des Ablaufschachts keine Flüssigkeit befindet, ist der dem Gas entgegengebrachte Widerstand im Bereich des Ablaufschachts kleiner, so dass das Gas bevorzugt über den Ablaufschacht aufsteigt, wodurch der Austausch zwischen Gas und Flüssigkeit verschlechtert wird. Die Höhe des Flüssigkeitsstandes auf dem Boden kann durch Flüssigkeitswehre am Ablaufschacht eingestellt werden. Erst wenn die Höhe des Flüssigkeitswehrs erreicht wird, kann die Flüssigkeit vom Boden durch den Ablaufschacht ablaufen.

Aus GB-A 2 002 650 sind Böden für eine Stoffaustauschkolonne bekannt, die mehrere Ablaufschächte aufweisen, wobei diese jeweils mit einem Wehr zur Einstellung des Flüssigkeitsniveaus auf dem Boden versehen sind. Die Ablaufschächte der einzelnen Böden sind übereinander angeordnet und damit die aus dem Ablaufschacht ablaufende Flüssigkeit nicht direkt in den darunter liegenden Ablaufschacht strömt, befindet sich unterhalb der Ablauföffnung des Ablaufschachts ein Umlenkblech, mit dem die ablaufende Flüssigkeit in den Bereich zwischen zwei Ablauföffnungen des unteren Bodens umgelenkt wird. Hierbei wird jedoch nur eine Umlenkung erzielt, ein Flüssigkeitsverschluss des Ablaufschachts lässt sich nicht erzielen.

DE-A 27 18 858 beschreibt eine Kolonne mit Böden, die jeweils einen Ablaufschacht aufweisen, wobei aufeinanderfolgende Böden jeweils um 180° zueinander gedreht sind. Durch die gedrehte Anordnung der Böden wird eine Querströmung auf dem Boden erzeugt. Für den Gasdurchtritt sind Öffnungen vorgesehen, die unterhalb des Ablaufschachts des oberen Bodens mit einer Abdeckplatte verschlossen sind, damit die ablaufende Flüssigkeit nicht direkt durch die Gasdurchtrittsöffnungen vom Boden abfließt.

Siebböden, die jeweils tieferliegende Bereiche aufweisen, aus denen die Flüssigkeit auf den darunter liegenden Boden abläuft und höherliegende Bereiche, mit Öffnungen durch die das aufsteigende Gas strömt, sind aus US 2,767,966 bekannt. Die Böden sind dabei jeweils so übereinander angeordnet, dass die tieferliegenden Bereiche des oberen Bodens über den höherliegenden Bereichen des unteren Bodens positioniert sind.

Aus US 4,273,618 sind Böden bekannt, die jeweils einen Ablaufschacht und einen Sammler aufweisen. Die Böden sind in einer Kolonne so angeordnet, dass jeweils ein Ablaufschacht oberhalb eines Sammlers endet, so dass die durch den Ablaufschacht ablaufende Flüssigkeit zunächst in den Sammler strömt und sobald der Flüssigkeitsstand die Höhe des den Sammler umgebenden Wehrs erreicht, läuft die Flüssigkeit aus dem Sammler auf den Boden.

Bei keinem der aus GB-A 2 002 650, DE-A 27 18 858, US 2,767,966 und US 4,274618 bekannten Böden ist ein Flüssigkeitsverschluss möglich, mit dem sich eine Bypass-Strömung des Gases durch die Ablaufschächte verhindern lässt.

Dokumente DE10257915, DE102012204436 und DE10332758 offenbaren Böden mit Ablaufschächten. Unterhalb der Ablaufschächte sind Ablauftassen angeordnet. Insbesondere bei Einsatz von Querstromböden in Stoffaustauschkolonnen, die bei der Herstellung von (Meth)acrylmonomeren eingesetzt werden, kann es zu einer unerwünschten Polymerisation kommen, die sich auch durch Polymerisationsinhibitoren nicht unterdrücken lässt. Insbesondere bei längeren Betriebsdauern kommt es zu einer verstärkten Ausbildung von unerwünschtem Polymerisat. Dies ist insbesondere deshalb von Nachteil, weil der Betrieb der Stoffaustauschkolonne regelmäßig unterbrochen werden muss, um das gebildete Polymerisat, das die Gasdurchtrittsöffnungen des Bodens ganz oder teilweise verschließen kann, zu entfernen.

Aufgabe der vorliegenden Erfindung ist es daher, einen Querstromboden bereitzustellen, der in einer Stoffaustauschkolonne eingesetzt werden kann und bei dessen Einsatz die aus dem Stand der Technik bekannten Nachteile behoben oder zumindest reduziert werden. Weiterhin ist es Aufgabe der Erfindung, eine Stoffaustauschkolonne bereitzustellen, die über einen längeren Zeitraum betrieben werden kann, ohne dass sich unerwünschte Ablagerungen bilden. Gelöst wird die Aufgabe durch einen Querstromboden für eine Stoffaustauschkolonne, in der ein Gas im Gegenstrom zu einer Flüssigkeit geführt wird, wobei der Boden Durchtrittsöffnungen für das Gas und mindestens zwei Ablaufschächte aufweist, wobei die Ablaufschächte über die Oberseite des Querstrombodens hinausragen und unterhalb jedes Ablaufschachts eine Auffangtasse angeordnet ist, wobei der Ablaufschacht in die Auffangtasse hineinragt, die minimale horizontale Querschnittsfläche der Auffangtasse 1,2 bis 4 mal größer ist als die horizontale Querschnittsfläche des Ablaufschachts am Auslass und wobei die Auffangtasse eine umlaufende Wandung mit einem Überlauf aufweist und wobei die Auffangtasse eine umlaufende Wandung mit einem Überlauf aufweist und wobei zur Bildung des Überlaufs Öffnungen in der Wandung der Auffangtasse vorgesehen sind oder die umlaufende Wandung der Auffangtasse zur Bildung des Überlaufs im Bereich des Überlaufs eine geringere Höhe aufweist.

Überraschenderweise hat sich gezeigt, dass bei einer Bodengestaltung derart, dass unterhalb jedes Ablaufschachts eine Auffangtasse angeordnet ist, in die der Ablaufschacht hineinragt und die eine umlaufende Wandung mit einem Überlauf aufweist die Bildung von Ablagerungen reduziert werden kann. Insbesondere beim Einsatz der Böden in einer Kolonne, die bei der Herstellung von (Meth)acrylmonomeren eingesetzt wird, lässt sich die Bildung von unerwünschtem Polymerisat reduzieren oder verhindern.

Durch die Verwendung der Auffangtasse, in die der Ablaufschacht hineinragt wird ein Flüssigkeitsverschluss realisiert, durch den eine Bypass-Strömung des Gases durch den Ablaufschacht verhindert wird. Der in die Auffangtasse hineinragende Ablaufschacht hat die Funktion eines Siphons. Um die Funktion des Siphons sicherzustellen ist die Auffangtasse so gestaltet, dass auch die den Überlauf aufweisende Wandung der Auffangtasse so gestaltet ist, dass der Flüssigkeitsstand in der Auffangtasse so hoch ist, dass der Ablaufschacht in die Flüssigkeit eintaucht, bevor Flüssigkeit aus der Auffangtasse über den Überlauf abfließen kann.

Die Durchtrittsöffnungen für das Gas können jede beliebige, dem Fachmann bekannte Geometrie aufweisen, wie sie für Durchtrittsöffnungen in Querstromböden geeignet ist. So können die Durchtrittsöffnungen zum Beispiel in Form von Löchern mit beliebiger Form ausgebildet sein. Bevorzugt sind die Durchtrittsöffnungen in diesem Fall rechteckig mit der langen Seite quer zur Hauptströmungsrichtung der Flüssigkeit auf dem Boden und der Boden ist in Form eines Siebbodens mit Ablaufschächten ausgebildet. Neben einer rechteckigen Gestaltung ist selbstverständlich auch jede andere Form denkbar, zum Beispiel in Form eines gebogenen Schlitzes, eines Ovals oder eines Kreises. Die Durchtrittsöffnungen können sowohl geordnet, zum Beispiel in parallelen oder axialen Reihen oder auch ungeordnet auf dem Boden angeordnet sein. Im Fall von gebogenen Schlitzen sind die Durchtrittsöffnungen vorzugsweise konzentrisch um den Mittelpunkt des Bodens angeordnet.

Alternativ zu einfachen Löchern als Durchtrittsöffnungen für das Gas ist es auch möglich, die Durchtrittsöffnungen als Ventile oder als Kamine mit einer Haube zu gestalten. Im Fall von Ventilen weist jede Durchtrittsöffnung einen Ventildeckel auf, der durch das durchströmende Gas angehoben wird. Hierdurch wird vermieden, dass bei einer zu geringen Gasströmung Flüssigkeit vom Boden durch die Ventile ablaufen kann. Wenn die Durchtrittsöffnungen mit einem Kamin mit Haube versehen sind, entspricht die Gestaltung der Durchtrittsöffnungen denen, wie sie auf Glockenböden oder Tunnelböden eingesetzt werden. Die Haube umschließt dabei den Kamin derart, dass durch die Durchtrittsöffnungen strömendes Gas in die Flüssigkeit auf dem Boden umgelenkt wird.

Besonders bevorzugt ist es, Glockenböden vom Typ Thormann® einzusetzen, bei denen die Durchtrittsöffnungen als rechteckige Glocken mit Ablenkblechen für das Gas ausgebildet sind.

In einer bevorzugten Ausführungsform der Erfindung ist der Überlauf auf der vom Mantel der Stoffaustauschkolonne abweisenden Seite der Auffangtasse angeordnet. Dies hat den Vorteil, dass sich im Bereich zwischen Mantel und Ablauftasse ein niedrigerer Flüssigkeitsstand einstellt, was weiterhin zur Folge hat, dass ein stärkerer Mitriss von Flüssigkeit erzeugt wird, der die darüber liegenden Bereiche nass hält. Da an mit Flüssigkeit benetzten Stellen kein Polymer entsteht, kann hierdurch die Ausbildung von Ablagerungen vermindert oder sogar verhindert werden. Im Folgenden wird auch der Begriff "Sprühschatten" verwendet, um den Bereich zu bezeichnen, der nicht von mit der Gasströmung in Form von Tröpfchen mitgerissener Flüssigkeit benetzt wird.

Besonders bevorzugt ist es, wenn alle Überläufe so ausgerichtet sind, dass die Mittelachse jedes Überlaufs senkrecht zur Mittelachse des Bodens ausgerichtet ist. Die vom Mantel der Stoffaustauschkolonne abweisende Seite der Auffangtasse ist dabei unabhängig von der Querschnittsform der Auffangtasse der Bereich, der in Richtung der Mittelachse des Bodens weist. Unabhängig von der Breite des Überlaufs bedeutet dies, dass die Mitte des Überlaufs in Richtung der Mittelachse des Bodens weist. Die Mittelachse des Bodens ist dabei eine Durchmesserlinie, die so ausgerichtet ist, dass die parallel zur Oberfläche des Bodens verlaufende Mittelachse des am weitesten von der Mittelachse des Bodens entfernt liegenden Überlaufs senkrecht zur Mittelachse des Bodens verläuft. Hierbei ist es besonders bevorzugt, wenn alle Ablaufschächte und damit alle Überläufe auf einer Seite der Mittelachse des Bodens angeordnet sind. Bevorzugt sind die Überläufe dabei so ausgerichtet, dass die Mittelachsen aller Überläufe parallel zueinander verlaufen. Als Mittelachse eines Überlaufs wird dabei die parallel zur Oberfläche des Bodens verlaufende Linie verstanden, die durch die Mitte des Überlaufs verläuft und üblicherweise eine Symmetrielinie der den Überlauf bildenden oberen Kante der Auffangtasse ist.

Bei einer Gestaltung der Ablaufschächte in Form von Langlöchern, die jeweils parallel verlaufen, so dass die Ablaufschächte und die Auffangtassen ebenfalls eine langgestreckte Querschnittsform aufweisen, ist es vorteilhaft, wenn die Überläufe so angeordnet sind, dass diese jeweils in Richtung der Mittelachse des Bodens ausgerichtet sind.

Bei einer rechteckigen oder quadratischen Querschnittsform der Auffangtasse bildet besonders bevorzugt eine Wand des Quadrats oder Rechtecks den Überlauf. Bei einer ovalen Querschnittsform mit parallel verlaufenden geraden Seiten und gebogenen Seiten, die die geraden Seiten verbinden, bildet vorzugsweise eine der gebogenen Seiten den Überlauf.

Wenn die Auffangtasse eine kreisförmige Querschnittsform aufweist, können bis zu 80% der Wandung der kreisförmigen Auffangtasse den Überlauf bilden. Bevorzugt bilden maximal 50% der Wandung der kreisförmigen Auffangtasse den Überlauf und besonders bevorzugt maximal 30%. Die Ausrichtung des Überlaufs wird dabei vorzugsweise so gewählt, dass wie vorstehend bereits dargelegt, der Mittelpunkt des Überlaufs in Richtung des Mittelpunkts des Bodens weist. Unter der "Querschnittsform" der Auffangtasse wird im Rahmen der vorliegenden Erfindung die Querschnittsform bei einem horizontalen Schnitt durch die Auffangtasse verstanden. Hierdurch entspricht die Querschnittsform im Allgemeinen auch der Form der Auffangtasse, die durch die Draufsicht auf die Auffangtasse gegeben ist. Bei nicht vertikal verlaufenden Wandungen der Auffangtasse kann sich die Querschnittsform allerdings vom Boden der Auffangtasse bis zum oberen Rand der Wandungen ändern. Im Allgemeinen wird jedoch auch in diesem Fall die Querschnittsform gleich bleiben und sich nur die Größe der Querschnittsfläche ändern.

Erfindungsgemäß wird der Überlauf, über den die Flüssigkeit aus der Auffangtasse abläuft durch Öffnungen in der Wandung der Auffangtasse, die als Überlauf wirken, realisiert, oder weist die umlaufende Wandung der Auffangtasse zur Bildung des Überlaufs im Bereich des Überlaufs eine geringere Höhe auf. Die Höhe des Überlaufs wird dabei vorzugsweise so gewählt, dass die Wandung im Bereich des Überlauf so hoch ist, dass der untere Abschluss des Ablaufschachts unterhalb der Oberkante der Ablauf tasse über ihren gesamten Umfang liegt.

In einer Ausführungsform der Erfindung verläuft die umlaufende Wandung senkrecht zum Boden der Auffangtasse. Dies hat den Vorteil, dass die Auffangtasse auch an ihrem oberen Ende den von der Gasströmung durchströmten Querschnitt nicht weiter verengt als am Boden. Die Gasströmung wird somit zwischen dem Boden der Auffangtasse und deren oberem Ende nicht weiter beschleunigt. Dies hat den Vorteil, dass keine aus dem Ablaufschacht abfließende Flüssigkeit mit der Gasströmung mitgerissen wird, zusätzlich wird auch der Bereich, der mit Flüssigkeit benetzt wird, die durch das durch den Boden strömende Gas aufgesprüht wird, nicht weiter verkleinert, so dass ein möglichst großer Bereich mit Flüssigkeit benetzt wird, so dass die Bildung von Polymer und damit das Entstehen von Ablagerungen verhindert wird.

Wenn die Wandungen der Auffangtassen eine Neigung aufweisen, nimmt die Querschnittsfläche der Auffangtasse vom Boden in Richtung des Oberen Randes der Wandungen der Auffangtasse zu. Der Winkel, mit dem die Wandungen zur Vertikalen geneigt sind, ist dabei vorzugsweise kleiner als 45°, mehr bevorzugt kleiner als 30° und insbesondere kleiner als 10°. Ganz besonders bevorzugt ist es jedoch, wenn die Wandungen der Auffangtasse parallel zur Vertikalen verlaufen, so dass der Winkel 0° beträgt.

Die Querschnittsform der Auffangtasse entspricht in einer Ausführungsform der Querschnittsform des Auffangschachts. In einer Ausführung der Erfindung ist die Auffangtasse zentrisch unter dem Auffangschacht angeordnet, so dass der Abstand der Wandung des Ablaufschachts im Bereich der Ablauföffnung zur Wandung des Ablaufschachts an jeder Stelle gleich groß ist. Besonders bevorzugt ist es allerdings, wenn der Abstand zwischen der Wandung des Ablaufschachts im Bereich der Ablauföffnung und der Wandung der Auffangtasse im Bereich des Überlaufs größer ist als der Abstand zur Wandung der Auffangtasse außerhalb des Überlaufs. Dies lässt sich zum Beispiel bei einem quadratischen Ablaufschacht dadurch realisieren, dass die Auffangtasse eine rechteckige Querschnittsform aufweist oder bei einem kreisförmigen Ablaufschacht durch eine ovale Auffangtasse. Alternativ könnte unabhängig von der Querschnittsform des Ablaufschachts eine kreisförmige Auffangtasse vorgesehen sein, wobei diese zum Mittelpunkt des Ablaufschachts exzentrisch angeordnet ist. Grundsätzlich können sowohl Ablaufschacht als auch Auffangtasse jeweils unabhängig voneinander eine beliebige Querschnittsform aufweisen, wobei die Auffangtasse jeweils so in Bezug auf den Ablaufschacht angeordnet ist, dass der Abstand zwischen der Wandung des Ablaufschachts und dem Überlauf größer ist als der Abstand zwischen der Wandung des Ablaufschachts und der Wandung der Auffangtasse außerhalb des Überlaufs.

Um den Einfluss des Ablaufschachts auf die Gasströmung zu minimieren, ist es bevorzugt, wenn der Ablaufschacht eine Querschnittsverengung aufweist, so dass die horizontale Querschnittsfläche des Ablaufschachts am Einlass größer ist als die horizontale Querschnittsfläche am Auslass. Durch die Verkleinerung der Querschnittsfläche des Ablaufschachts ist es möglich, die Auffangtasse so zu gestalten, dass diese eine Querschnittsfläche aufweist, die nicht größer ist als die Querschnittsfläche des Ablaufschachts oberhalb der Querschnittsverengung. Auch dies hat den Vorteil, dass die Bereiche minimiert werden, die durch aufsprühende Flüssigkeit nicht benetzt werden, so dass die Bildung von Ablagerungen durch ausfallendes Polymer minimiert wird.

Das Verhältnis der horizontalen Querschnittsfläche am Einlass zur horizontalen Querschnittsfläche am Auslass des Ablaufschachts liegt vorzugsweise im Bereich von 1 : 1 bis 4 : 1, mehr bevorzugt im Bereich von 1,5 : 1 bis 3 : 1 und insbesondere im Bereich von 1,8 : 1 bis 2,5 : 1. Um die Querschnittsverengung zu realisieren, ist es möglich, den Ablaufschacht trichterförmig zu gestalten, so dass alle den Ablaufschacht bildenden Wandungen eine Neigung zur Mitte des Ablaufschachts aufweisen. Alternativ, insbesondere bei einem Ablaufschacht mit nicht kreisförmigem Querschnitt, ist es auch möglich, nur eine Wandung oder bevorzugt zwei gegenüberliegende Wandungen mit einer Neigung in Richtung zur Mitte des Ablaufschachts zu versehen, so dass sich die Länge des Ablaufschachts parallel zu den Wandungen mit Neigung nicht ändert und nur quer zur Ausrichtung der Wandungen mit Neigung die Breite des Ablaufschachts abnimmt.

Erfindungsgemäß ist die minimale horizontale Querschnittsfläche der Auffangtasse 1,2 bis 4 mal größer ist als die horizontale Querschnittsfläche des Ablaufschachts am Auslass, bevorzugt 1,5 bis 3 mal größer und insbesondere 1,8 bis 2,5 mal größer. Weiterhin ist es bevorzugt, wenn die horizontale Querschnittsfläche der Auffangtasse 0,5 bis 2 mal so groß ist wie die horizontale Querschnittsfläche des Ablaufschachts am Einlass, bevorzugt 0,7 bis 1,5 mal so groß und insbesondere 0,8 bis 1,2 mal so groß.

Unabhängig von der Art der Querschnittsverengung des Ablaufschachts ist es bevorzugt, wenn der Ablaufschacht einen oberen Bereich mit einem konstanten Querschnitt aufweist, einen mittleren Bereich, in dem die Querschnittsfläche abnimmt und einen unteren Bereich, der wiederum einen konstanten Querschnitt aufweist. Die Querschnittsfläche des oberen Bereichs mit konstantem Querschnitt entspricht dabei der Fläche des Einlasses des Ablaufschachts und die Querschnittsfläche des unteren Bereichs der Querschnittsfläche am Auslass. Besonders bevorzugt verlaufen die Wandungen im oberen Bereich und im unteren Bereich vertikal.

Neben einer Gestaltung mit drei Bereichen ist es allerdings auch möglich, nur zwei Bereiche vorzusehen, wobei der obere Bereich eine konstante Querschnittsfläche und der untere Bereich eine Querschnittsverengung aufweist oder der obere Bereich die Querschnittsverengung und der untere Bereich eine konstante Querschnittsfläche. Auch ist es möglich, die Querschnittsverengung über die gesamte Länge des Ablaufschachts zu realisieren, so dass dieser nur den Bereich mit Querschnittsverengung aufweist.

Bei einer nicht kreisförmigen Gestaltung des Ablaufschachts ist es besonders bevorzugt, wenn der Ablaufschacht symmetrisch zu einer vertikal mittig im Ablaufschacht verlaufenden Symmetrieebene ausgebildet ist. Wenn in diesem Fall der Ablaufschacht eine Querschnittsverkleinerung vom Einlauf in Richtung Auslauf aufweist, so wird diese vorzugsweise so realisiert, dass die Wandungen, die parallel zur Symmetrieebene verlaufen eine Neigung in Richtung der Symmetrieebene aufweisen. Hierbei verlaufen die Wandungen vorzugsweise im oberen Bereich des Ablaufschachts vertikal, haben im mittleren Bereich die Neigung und verlaufen im unteren Bereich wieder vertikal. Wie vorstehend beschrieben besteht jedoch auch hier die Möglichkeit, die Wandungen so zu gestalten, dass nur der Bereich oberhalb der Querschnittsverengung oder nur der Bereich unterhalb der Querschnittsverengung eine konstante Querschnittsfläche aufweist oder aber die Querschnittsfläche über den gesamten Ablaufschacht von oben nach unten abnimmt. Unabhängig von der Gestaltung des Ablaufschachts mit oder ohne Abschnitten mit konstant bleibender Querschnittsfläche ist es bevorzugt, wenn bei einer Gestaltung symmetrisch zu einer vertikal mittig im Ablaufschacht verlaufenden Symmetrieebene die parallel zur Symmetrieebene verlaufenden Wandungen des Ablaufschachtes einen Bereich aufweisen, in dem die Wandung in einem Winkel zwischen 10 und 80° geneigt zur Vertikalen verläuft, um die Querschnittsverengung auszubilden. Bevorzugt ist die Wandung in einem Winkel zwischen 20 und 60° geneigt zur Vertikalen und insbesondere im Bereich von 30 bis 50°. Durch kleinere Neigungen wird die Gasströmung weniger stark abgelenkt, so dass auch hierdurch der Sprühschatten verkleinert wird.

Die Ablaufschächte sind vorzugsweise so auf einem Querstromboden angeordnet, dass zumindest zwischen einem Teil der Ablaufschächte und der Stoffaustauschkolonne ein ausreichender Abstand ist, dass mindestens eine Durchtrittsöffnung für das Gas zwischen dem Ablaufschacht und dem äußeren Rand des Querstrombodens ausgebildet sein kann.

In einer bevorzugten Ausführungsform sind alle Ablaufschächte eines Bodens so positioniert, dass der Abstand zwischen der durch den Boden ragenden Wandung des Ablaufschachtes und dem äußeren Rand des Bodens an jeder Stelle mindestens so groß ist, dass mindestens eine Durchtrittsöffnung im Boden ausgebildet sein kann. Auf diese Weise wird sichergestellt, dass auch die Flüssigkeit, die sich zwischen dem Ablaufschacht und der Wandung der Kolonne befindet, vom aufsteigenden Gas durchströmt wird. Hierdurch wird der Kontakt zwischen der Flüssigkeit und dem Gas weiter intensiviert. Durch die Durchtrittsöffnung zwischen dem äußeren Rand des Bodens und dem Ablaufschacht wird auch an dieser Stelle mit der Gasströmung Flüssigkeit mitgerissen, so dass auch im Bereich zwischen dem Rand des Bodens und dem Ablaufschacht die Wandungen der Kolonne mit Flüssigkeit benetzt werden und sich keine Polymerablagerungen bilden.

Die Gesamtfläche der Querschnittsflächen der Zuläufe aller Ablaufschächte eines Querstrombodens liegt vorzugsweise im Bereich von 0,2 bis 5%, mehr bevorzugt im Bereich von 0,2 bis 4% und insbesondere im Bereich von 0,5 bis 2%.

Eine geeignete Gestaltung und Anordnung der Ablaufschächte eines Querstrombodens ist zum Beispiel in WO-A 2013/139590 beschrieben.

Eine erfindungsgemäße Stoffaustauschkolonne zur Durchführung eines Stoffaustauschprozesses umfasst mindestens zwei der vorstehend beschriebenen Querstromböden. Die Querstromböden sind dabei so angeordnet, dass die Flüssigkeit über die Ablaufschächte des oberen Bodens auf den Boden strömt, dann über den Boden fließt und durch die Ablaufschächte des Bodens auf einen darunter liegenden Boden abläuft oder beim untersten Boden der Stoffaustauschkolonne im Sumpf der Kolonne aufgefangen wird. Um den Gasaustausch zu gewährleisten sind in jedem Boden die Durchtrittsöffnungen für das Gas ausgebildet. Die Anzahl der Öffnungen in jedem Boden und die Gesamtquerschnittsfläche der Öffnungen wird so gewählt, dass der Druck des Gases unterhalb jedes Bodens der Stoffaustauschkolonne ausreichend ist, um zu verhindern, dass Flüssigkeit durch die Durchtrittsöffnungen für das Gas vom Boden ablaufen kann. Bevorzugt ist es jedoch, die Durchtrittsöffnungen mit einem nach oben ragenden Rand und einer darüber liegenden Haube als Glocken zu gestalten, durch die verhindert wird, dass Flüssigkeit vom Boden durch die Durchtrittsöffnungen ablaufen kann.

Um die Verweilzeit der Flüssigkeit auf jedem Boden und damit den Kontakt zwischen Gas und Flüssigkeit auf jedem Boden zu maximieren, ist es bevorzugt, wenn alle Ablaufschächte eines Querstrombodens in der gleichen Hälfte des Bodens angeordnet sind und jeweils zwei übereinanderliegende Querstromböden so angeordnet sind, dass die Ablaufschächte des oberen Querstrombodens oberhalb der Hälfte des unteren Querstrombodens enden, in der keine Ablaufschächte angeordnet sind. Auf diese Weise läuft die Flüssigkeit auf einer Hälfte auf den Querstromboden und in der anderen Hälfte vom Querstromboden ab, nachdem die Flüssigkeit auf dem Querstromboden von der einen Hälfte in die andere Hälfte geflossen ist.

In einer bevorzugten Ausführungsform wird die erfindungsgemäße Stoffaustauschkolonne zur thermischen Trennung eines (Meth)acrylmonomere enthaltenden Gemischs, bei dem eine Flüssigkeit und ein Gasstrom im Gegenstrom durch die Stoffaustauschkolonne geleitet werden und die (Meth)acrylmonomere entweder im Gasstrom oder in der Flüssigkeit enthalten sind, eingesetzt.

(Meth)acrylmonomere im Rahmen der vorliegenden Erfindung bedeuten Acrylmonomere und/oder Methacrylmonomere.

Acrylmonomere bedeuten Acrolein, Acrylsäure und/oder Ester der Acrylsäure. Methacrylmonomere bedeuten Methacrolein, Methacrylsäure und/oder Ester der Methacrylsäure.

Insbesondere umfassen die (Meth)acrylmonomere im Rahmen der vorliegenden Erfindung Hydroxyethylacrylat, Hydroxyehtylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, iso-Butylmethacrylat, n-Butylmethacrylat, tert.-Butylacrylat, tert.-Butylmethacrylat, Ethylacrylat, Ethylmethacrylat, 2-Ethylhexylacrylat, 2-Ethylhexylmethacrylat, N,N-Dimethylaminoethylacrylat und N,N-Dimethylaminoethylmethacrylat.

Großtechnisch werden (Meth)acrolein und (Meth)acrylsäure vorwiegend durch katalytische Gasphasenoxidation geeigneter C₃-/C₄-Vorläuferverbindungen (oder von Vorläuferverbindungen derselben) hergestellt. Im Fall von Acrolein und Acrylsäure werden als solche Vorläuferverbindungen bevorzugt Propen und Propan verwendet. Im Fall der Methacrylsäure und des Methacroleins sind iso-Buten und iso-Butan die bevorzugten Vorläuferverbindungen.

Neben Propen, Propan, iso-Buten und iso-Butan eignen sich als Ausgangsstoffe jedoch auch andere 3 beziehungsweise 4 Kohlenstoffatome enthaltende Verbindungen wie iso-Butanol, n-Propanol oder Vorläuferverbindungen derselben, beispielsweise der Methylether von iso-Butanol. Acrylsäure kann auch durch gasphasenkatalytische Oxidation von Acrolein erzeugt werden, Methacrylsäure entsprechend durch gasphasenkatalytische Oxidation von Methacrolein.

Im Rahmen der Herstellverfahren werden Produktgasgemische erhalten, aus denen die (Meth)acrylsäure beziehungsweise das (Meth)acrolein abgetrennt werden müssen.

Ester der (Meth)acrylsäure sind zum Beispiel durch direkte Umsetzung von (Meth)acrylsäure und/oder (Meth)acrolein mit den entsprechenden Alkoholen erhältlich. Allerdings fallen auch in diesem Fall Produktgemische an, aus denen die (Meth)acrylsäureester abgetrennt werden müssen.

Unabhängig vom Herstellverfahren und von den hergestellten (Meth)acrylmonomeren werden die (Meth)acrylmonomere in einer Stoffaustauschkolonne, vorzugsweise durch ein thermisches Trennverfahren, abgetrennt. Bei dem thermischen Trennverfahren werden ein Flüssigkeitsstrom und ein Gasstrom im Gegenstrom durch die Stoffaustauschkolonne geführt, wobei das Gas in intensiven Kontakt mit der Flüssigkeit kommt. Für den intensiven Kontakt enthält die Stoffaustauschkolonne mehrere der erfindungsgemäßen Querstromböden, auf denen sich Flüssigkeit befindet, durch die das aufsteigende Gas durchperlt.

Besonders bevorzugt ist das thermische Trennverfahren eine Kondensation, wenn die (Meth)-acrylmonomere im Gasstrom enthalten sind, und eine Rektifikation, wenn die (Meth)acrylmonomere in der Flüssigkeit enthalten sind. Auf diese Weise werden die (Meth)acrylmonomere immer als Flüssigkeit am Sumpf der Stoffaustauschkolonne abgezogen.

Ausführungsformen der Erfindung sind in den Figuren dargestellt und werden in der nachfolgenden Beschreibung näher erläutert.

Es zeigen:
- Figur 1: eine Draufsicht auf einen Querstromboden,
- Figur 2: eine vertikale Schnittansicht durch einen Ablaufschacht in einer ersten Ausführungsform,
- Figur 3: eine vertikale Schnittansicht durch einen Ablaufschacht in einer zweiten Ausführungsform,
- Figur 4: eine vertikale Schnittansicht durch einen Ablaufschacht in einer dritten Ausführungsform,
- Figur 5: eine vertikale Schnittansicht durch den Ablaufschacht gemäß Figur 4 in einer um 90° gedrehten Darstellung,
- Figur 6: einen Längsschnitt durch einen Abschnitt einer Stoffaustauschkolonne.

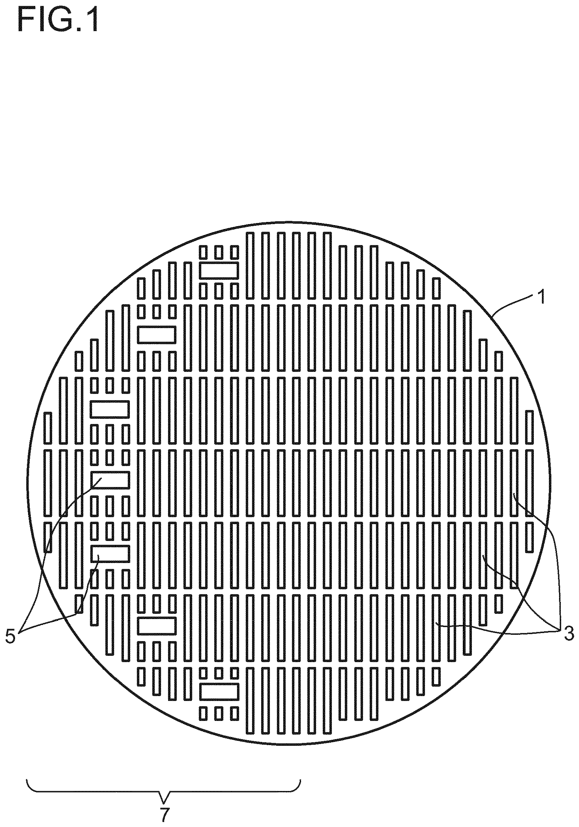

Figur 1 zeigt eine Draufsicht auf einen Querstromboden.

Ein Querstromboden 1, der in einer Stoffaustauschkolonne eingesetzt werden kann, weist Durchtrittsöffnungen 3 für ein Gas und Ablaufschächte 5, durch die die Flüssigkeit vom Querstromboden 1 ablaufen kann, auf.

Im Betrieb der Stoffaustauschkolonne gelangt Flüssigkeit über die Ablaufschächte eines oberhalb liegenden Bodens auf den Querstromboden 1. Die Flüssigkeit strömt in Richtung der Ablaufschächte 5, wobei gleichzeitig Gas durch die Durchtrittsöffnungen 3 strömt. Die Durchtrittsöffnungen 3 für das Gas sind dabei vorzugsweise als Glocken mit einem nach oben weisenden Rand und einer darüber angeordneten Haube ausgebildet. Hierdurch strömt das Gas durch die Durchtrittsöffnung, wird an der Haube nach unten abgelenkt und durch die die Durchtrittsöffnung umgebende Flüssigkeit geleitet. Die Hauben sind dabei so gestaltet, dass diese in die Flüssigkeit hineinragen. Durch die Gestaltung der Durchtrittsöffnungen 3 als Glocken kann keine Flüssigkeit durch die Durchtrittsöffnungen 3 ablaufen. Die Flüssigkeit strömt so zu den Ablaufschächten 5 und läuft durch die Ablaufschächte 5 auf einen darunter liegenden Boden ab.

Alternativ zur Gestaltung der Durchtrittsöffnungen 3 als Glocken ist auch jede andere Gestaltung, die dem Fachmann bekannt ist, möglich. Die Durchtrittsöffnungen 3 werden dabei so gestaltet, dass im Betrieb keine Flüssigkeit durch die Durchtrittsöffnungen 3 ablaufen kann. Wenn die Durchtrittsöffnungen in Form von Löchern wie bei einem Siebboden gestaltet sind, muss die Gasströmung so eingestellt werden, dass keine Flüssigkeit durch die Durchtrittsöffnungen 3 ablaufen kann. Bevorzugt ist jedoch die Gestaltung als Glocken, zum Beispiel mit rechteckigem Querschnitt wie bei einem Thormann®-Boden.

Damit die Kontaktzeit zwischen Gas und Flüssigkeit ausreichend groß gehalten werden kann, sind die Ablaufschächte 5 eines Bodens vorzugsweise wie in Figur 1 dargestellt alle in einer Hälfte 7 des Querstrombodens 1 angeordnet. Innerhalb einer Stoffaustauschkolonne weisen alle Querstromböden vorzugsweise das gleiche Design auf, wobei jeweils zwei unmittelbar übereinander liegende Böden jeweils um 180° zueinander gedreht sind. Hierdurch fließt die Flüssigkeit des oberen Bodens in der Hälfte auf den Querstromboden 1, in der keine Ablaufschächte angeordnet sind. Die Flüssigkeit läuft dann in die andere Hälfte 7 des Querstrombodens, in der die Ablaufschächte 5 angeordnet sind und durch die Ablaufschächte 5 ab.

Um einen ausreichend hohen Flüssigkeitsstand auf dem Querstromboden 1 zu erhalten, ragen die Ablaufschächte 5 vorzugsweise über die Oberfläche des Querstrombodens 1 hinaus und bilden ein Wehr, über das die Flüssigkeit in den Ablaufschacht 5 strömt.

Die Durchtrittsöffnungen 3 können, wie hier dargestellt, als rechteckige Schlitze ausgebildet sein. In diesem Fall ist die lange Seite jedes Schlitzes vorzugsweise quer zur Strömungsrichtung der Flüssigkeit ausgerichtet. Neben der Gestaltung der Durchtrittsöffnungen 3 in Form von rechteckigen Schlitzen ist es auch möglich, die Durchtrittsöffnungen 3 als Langlöcher mit abgerundeten kurzen Seiten, oval, kreisförmig oder mit jeder beliebigen Querschnittsform auszuführen. Bevorzugt ist jedoch ein kreisförmiger Querschnitt oder wie hier dargestellt ein rechteckiger Querschnitt oder die Form eines Langlochs. Die einzelnen Durchtrittsöffnungen 3 können wie hier dargestellt in parallelen Reihen angeordnet sein. Alternativ ist jedoch auch eine radiale Anordnung oder eine Anordnung, bei der die Durchtrittsöffnungen 3 benachbarter parallelen Reihen zueinander versetzt sind, möglich. Auch jede andere Anordnung der Durchtrittsöffnungen 3, mit der ein ausreichender Kontakt von Gas und Flüssigkeit realisiert werden kann und bei der vermieden wird, dass Flüssigkeit durch die Durchtrittsöffnungen 3 abläuft, ist möglich.

Neben einer Gestaltung der Durchtrittsöffnungen als Löcher ist es auch möglich, die Durchtrittsöffnungen in Form von Ventilen oder Kaminen mit Haube zu gestalten. Bei einer Gestaltung als Kamin mit Haube entspricht die Form der Durchtrittsöffnungen der Form, wie sie auf Tunnelböden ausgeführt ist. Bei kreisförmigen Durchtrittsöffnungen mit Kamin und Haube entspricht die Gestaltung einem Glockenboden.

Wenn die Durchtrittsöffnungen 3 als Ventile ausgebildet sind, so befinden sich oberhalb der Öffnungen Ventildeckel, die von der Gasströmung angehoben werden, so dass das Gas durch den Boden in die darauf fließende Flüssigkeit strömen kann. Sobald die Gasströmung zu schwach ist oder unterbrochen wird, werden die Öffnungen von den Ventildeckeln geschlossen, so dass keine Flüssigkeit vom Boden durch die Öffnungen ablaufen kann.

Grundsätzlich ist jede Gestaltung möglich, die dem Fachmann bekannt ist und mit der Gas durch eine auf dem Boden strömende Flüssigkeit geleitet werden kann.

Erfindungsgemäß weist der Querstromboden 1 mehrere Ablaufschächte 5 auf. Hierdurch wird erreicht, dass die ablaufende Flüssigkeit über den gesamten Bereich, in dem die Ablaufschächte 5 angeordnet sind durch das Auftreffen auf die Flüssigkeit des unteren Querstrombodens Tröpfchen bildet und sprüht. Überraschenderweise führt dies zu einer Reduktion der Polymerisatbildung und der Bildung von Ablagerungen.

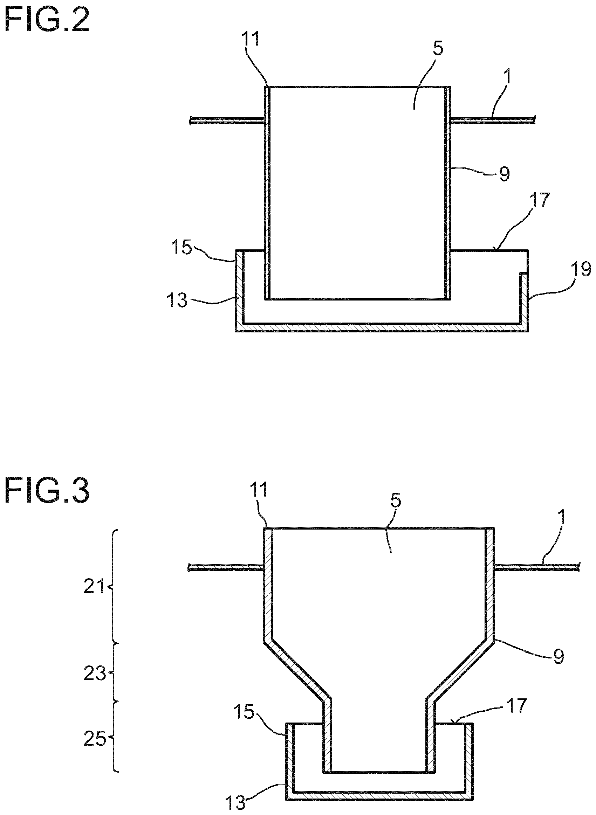

Geeignete Gestaltungen für die Ablaufschächte 5 sind in den Figuren 2 und 3 dargestellt.

Ein Ablaufschacht 5 weist eine umlaufende Wandung 9 auf, die vorzugsweise, wie hier dargestellt über den Querstromboden 1 nach oben hinausragt. Hierdurch wird ein Wehr 11 gebildet. Die Höhe des Wehres 11 bestimmt dabei die Höhe der Flüssigkeit auf dem Querstromboden 1. Je höher das Wehr 11 ist, umso höher muss der Flüssigkeitsstand sein, damit Flüssigkeit vom Querstromboden 1 über das Wehr 11 in den Ablaufschacht 5 fließen kann.

Unterhalb des Ablaufschachtes 5 befindet sich erfindungsgemäß eine Auffangtasse 13. Die Auffangtasse 13 weist eine umlaufende Wandung 15 auf, die so hoch ist, dass die Wandung 9 des Ablaufschachtes 5 unterhalb des oberen Randes 17 der umlaufenden Wandung 15 der Auffangtasse 13 endet. Im laufenden Betrieb führt dies dazu, dass sich Flüssigkeit in der Auffangtasse 13 sammelt und die Wandung 9 des Ablaufschachts 5 in die Flüssigkeit hineinragt. Hierdurch wird ein Flüssigkeitsverschluss erzeugt, durch den vermieden wird, dass Gas durch den Ablaufschacht nach oben entweichen kann. Das gesamte Gas muss somit durch die Durchtrittsöffnungen 3 strömen.

Auf einer Seite der umlaufenden Wandung 15 der Auffangtasse 13 ist ein Überlauf 19 ausgebildet. Der Überlauf 19 kann, wie in Figur 2 dargestellt realisiert sein, indem die umlaufende Wandung 15 im Bereich des Überlaufs 19 eine geringere Höhe aufweist. Alternativ ist es auch möglich, den Überlauf zum Beispiel in Form von Öffnungen in der umlaufenden Wandung 15 der Auffangtasse 13 zu gestalten.

Unabhängig von der Form des Überlaufs ist jedoch grundsätzlich darauf zu achten, dass die den Überlauf bildende Kante - hier der obere Abschluss des den Überlauf 19 bildenden Wandabschnitts - oberhalb des unteren Abschlusses der Wandung 9 des Ablaufschachts 5 liegt, um den Flüssigkeitsverschluss sicherzustellen. Wie vorstehend beschrieben, ist es bevorzugt, wenn der Überlauf 19 auf vom Mantel der Stoffaustauschkolonne weg weisenden Seite angeordnet ist.

Neben der Ausführungsform des Ablaufschachtes 5 mit konstantem Querschnitt, wie in Figur 2 dargestellt, ist es auch möglich, den Ablaufschacht 5 mit einer Querschnittsverengung zu gestalten. Hierbei ist es einerseits möglich, den Querschnitt über die gesamte Länge des Ablaufschachtes 5 zu verkleinern oder, wie in Figur 3 dargestellt, den Ablaufschacht in einen oberen Bereich 21 mit konstanter Querschnittsfläche, einen mittleren Bereich 23 mit abnehmender Querschnittsfläche und einem unteren Bereich mit konstanter Querschnittsfläche zu gestalten.

In der hier dargestellten Ausführungsform verläuft die Wandung des Ablaufschachts 5 im oberen Bereich 21 vertikal, so dass die Querschnittsfläche konstant bleibt. An den oberen Bereich 21 schließt sich der mittlere Bereich 23 an, in dem die Wandungen so geneigt sind, dass die Querschnittsfläche des Ablaufschachts 5 von oben nach unten abnimmt. Im unteren Bereich 25 verlaufen die Wandungen wieder vertikal nach unten, so dass die Querschnittsfläche hier ebenfalls konstant ist.

Besonders bevorzugt ist es, wenn der Ablaufschacht 5 symmetrisch zu einer vertikal verlaufenden Symmetrieebene ausgebildet ist. Bei einer Querschnittsverengung ist es dabei möglich, dass alle Wandungen des Ablaufschachts eine Neigung aufweisen, so dass der Ablaufschacht in alle Richtungen von oben nach unten hinsichtlich der Querschnittsfläche kleiner wird.

Alternativ und bevorzugt ist es jedoch, wenn bei einem rechteckigen Ablaufschacht 5 zwei Wandungen parallel vertikal über die gesamte Länge des Ablaufschachts 5 verlaufen und die beiden anderen gegenüberliegenden Wandungen einen geneigten Abschnitt zur Ausbildung der Querschnittsverkleinerung aufweisen. In diesem Fall hat der Ablaufschacht einen ersten Querschnitt, wie er in Figur 2 dargestellt ist und einen zweiten Querschnitt, der zum ersten Querschnitt um 90° gedreht ist, wie er in Figur 3 dargestellt ist.

Unabhängig von der Gestalt des Ablaufschachts 5 mit oder ohne Querschnittsverengung, weist die Auffangtasse 13 erfindungsgemäß immer einen Bereich auf, in dem der Überlauf 19 ausgebildet ist.

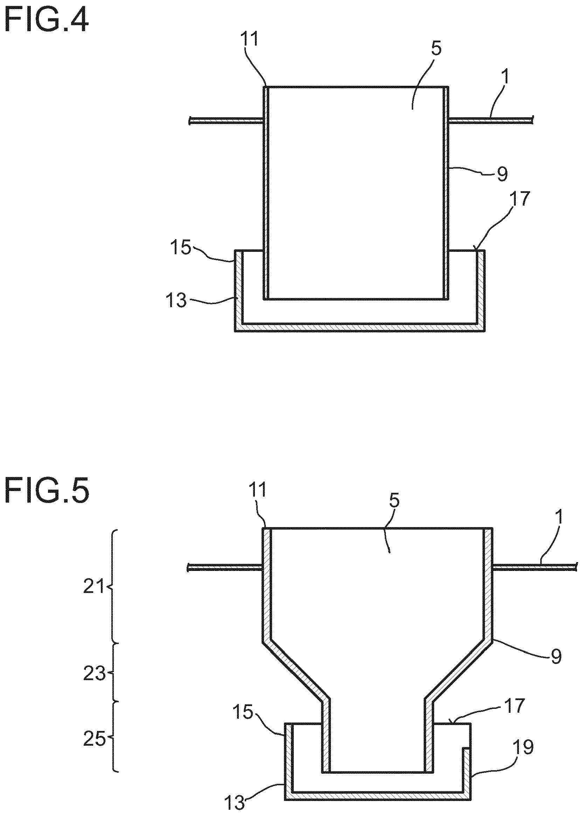

In den Figuren 4 und 5 ist eine weitere Ausführungsform für einen Ablaufschacht dargestellt. Hierbei sind die Schnittdarstellungen um 90° zueinander gedreht, so dass Figur 4 eine Schnittdarstellung senkrecht zur Mittelachse des Bodens zeigt und Figur 5 eine Schnittdarstellung parallel zur Mittelachse des Bodens.

Im Unterschied zu der in den Figuren 2 und 3 dargestellten Ausführungsform ist bei der in den Figuren 4 und 5 dargestellten Ausführungsform der Überlauf 19 der Auffangtasse 13 parallel zu einer Wandung mit Querschnittsverengung angeordnet. Dies hat den Vorteil, dass die Seite der Auffangtasse 13, die den Überlauf 19 aufweist, nicht über die Querschnittsfläche des Ablaufschachts am Einlass hinausragt. Hierdurch lässt sich der Sprühschatten verringern.

Auf den Seiten parallel zu den Seiten ohne Querschnittsverengung kann hierdurch ein minimaler Überstand der Auffangtasse 13 realisiert werden, wie aus Figur 5 entnehmbar ist. Durch die entsprechende Gestaltung von Ablaufschacht 9 und Auffangtasse 13 lässt sich ein minimaler Sprühschatten realisieren.

Wenn die Ablaufschächte wie in Figur 1 angeordnet sind, sind die Überläufe aller Auffangtassen vorzugsweise auf der gleichen Seite der Ablaufschächte, insbesondere zeigen diese zur Durchmesserlinie, die parallel zur langen Seite der Durchtrittsöffnungen 3 ausgerichtet ist. Wenn die Ablaufschächte einen kreisförmigen Querschnitt aufweisen oder in radialer Richtung ausgerichtet sind, zeigt die Mitte jedes Überlaufs vorzugsweise in Richtung des Mittelpunkts des Querstrombodens 1. Alternativ ist es jedoch auch bei kreisförmigen Ablaufschächten möglich, dass alle Überläufe 19 in die gleiche Richtung hin zu den Ablauföffnungen des unteren Bodens weisen.

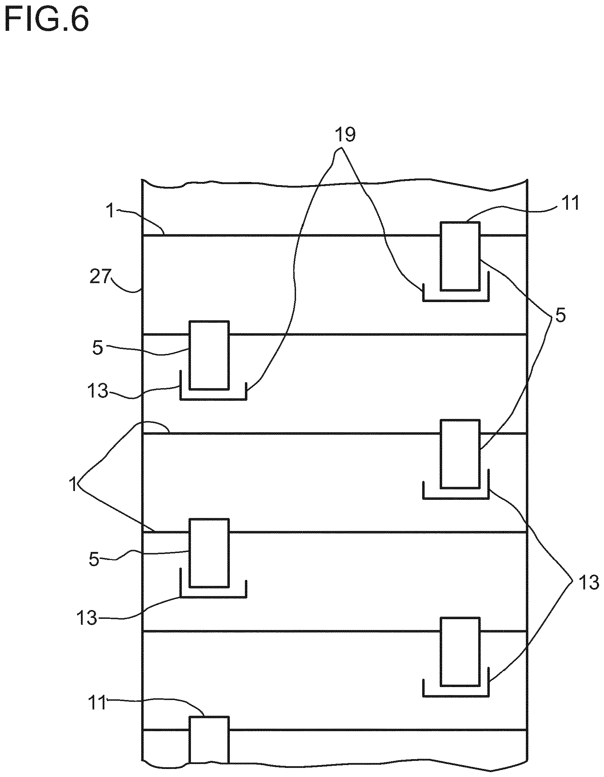

Figur 6 zeigt eine mögliche Anordnung von Querstromböden in einer Stoffaustauschkolonne. Hierzu ist lediglich ein Ausschnitt der Kolonne dargestellt. Die Anordnung der Böden setzt sich nach oben und nach unten entsprechend fort. Kopf und Sumpf der Stoffaustauschkolonne werden dabei so, wie es dem Fachmann bekannt ist, gestaltet. Am Kopf der Kolonne befindet sich dabei ein Gasauslass und am Sumpf der Kolonne ein Flüssigkeitsablass. Die Zufuhr in die Kolonne ist abhängig vom durchgeführten Stoffaustauschprozess. Bei einer Kondensation wird im unteren Bereich der Kolonne ein Gas zugeführt, dass in der Stoffaustauschkolonne aufsteigt wobei auf den einzelnen Böden schwerer siedende Flüssigkeit auskondensiert. Wenn der Stoffaustauschprozess eine Rektifikation ist, wird oben eine Flüssigkeit zugegeben und am Sumpf befindet sich eine Heizung, so dass die Flüssigkeit auf jedem Boden siedet, das Gas aufsteigt und nicht siedende Flüssigkeit nach unten abläuft. Im Fall einer Rektifikation ist neben einer Zugabe oben auch eine Zugabe der Flüssigkeit unten oder über einen Seitenzulauf möglich.

Auch bei einer Kondensation kann das Gas über einen Seitenzulauf zugegeben werden.

Um einen möglichst intensiven Kontakt von Gas und Flüssigkeit auf jedem Boden zu erhalten, sind die einzelnen Querstromböden 1 in der Stoffaustauschkolonne 27 jeweils um 180° zueinander gedreht angeordnet. Auf diese Weise liegen die Ablaufschächte 5 aufeinanderfolgender Böden jeweils auf der gegenüberliegenden Seite in der Stoffaustauschkolonne 1, so dass die Flüssigkeit auf jedem Boden von der Seite der Ablaufschächte 5 des oberen Bodens, durch die die Flüssigkeit auf den jeweiligen Querstromboden 1 gelangt, zu den Ablaufschächten 5 des Querstrombodens 1 fließen muss. Durch die in Figur 6 nicht dargestellten Durchtrittsöffnungen strömt das Gas von unten durch jeden Querstromboden, so dass aufgrund der Gasströmung verhindert wird, dass die Flüssigkeit durch die Durchtrittsöffnungen nach unten ablaufen kann. Die Flüssigkeit strömt zum Ablaufschacht 5, über das Wehr 11 in den Ablaufschacht 5 und wird in der Auffangtasse 13 aufgefangen. Aus der Auffangtasse 13 strömt die Flüssigkeit über den Überlauf 19 auf den darunter liegenden Querstromboden 1. Hierbei sind die Überläufe 19 vorzugsweise jeweils in Richtung der Ablaufschächte 5 des darunter liegenden Querstrombodens 1 ausgerichtet.

### Beispiele

### Vergleichsbeispiel

Es wird entsprechend dem in WO 2013/139590 beschriebenen Beispiel verfahren. Hierbei hat sich gezeigt, dass zwar wie in der WO 2013/139590 beschrieben nach 70 Tagen ununterbrochenem Betrieb noch keine Bildung von unerwünschtem Polymerisat in der Kondensationskolonne feststellbar war, sich eine solche Bildung jedoch nach 3 Monaten gezeigt hat, die eine Reinigung der Kolonne erforderlich gemacht hat.

### Beispiel 1

Bei dem in WO 2013/139590 beschriebenen Verfahren wurden die Ablaufschächte und Auffangtassen der Thormann®-Böden der Kondensationskolonne durch Ablaufschächte ersetzt, die eine rechteckige Querschnittsform aufwiesen und die eine Querschnittsverengung aufwiesen, wie in den Figuren 4 und 5 dargestellt, wobei die Querschnittsfläche am Einlass doppelt so groß wie die Querschnittsfläche am Auslass war. Die Auffangtasse hatte ebenfalls eine Querschnittsfläche, die doppelt so groß wie die Querschnittsfläche des Auslasses war. Die Auffangtassen hatten jeweils senkrecht nach oben ragende Seitenwände, wobei die zur Mittelachse des Bodens weisende Seitenwand niedriger war und so einen Überlauf gebildet hat. Der Überlauf war dabei unterhalb der Fläche des Einlasses positioniert und ragte nur seitlich an den nicht verengten Seiten über die Fläche des Ablaufschachts hinaus.

Jeder der Thormann®-Böden war mit 8 Ablaufschächten, die alle die gleiche Geometrie aufwiesen und die parallel zueinander ausgerichtet waren, ausgestattet.

Die Kolonne wurde wie in der WO 2013/139590 betrieben. Hierbei hat sich gezeigt, dass sich auch nach sechs Monaten noch keine Ablagerungen gebildet haben.

### Bezugszeichenliste

- 1: Querstromboden
- 3: Durchtrittsöffnung
- 5: Ablaufschacht
- 7: Hälfte des Querstrombodens mit Ablaufschächten 5
- 9: Wandung
- 11: Wehr
- 13: Auffangtasse
- 15: umlaufende Wandung
- 17: oberer Rand
- 19: Überlauf
- 21: oberer Bereich
- 23: mittlerer Bereich
- 25: unterer Bereich

## Patentansprüche

1. Querstromboden für eine Stoffaustauschkolonne (27), in der ein Gas im Gegenstrom zu einer Flüssigkeit geführt wird, wobei der Querstromboden (1) Durchtrittsöffnungen (3) für das Gas und mindestens zwei Ablaufschächte (5) aufweist, wobei die Ablaufschächte (5) über die Oberseite des Querstrombodens (1) hinausragen und unterhalb jedes Ablaufschachts (5) eine Auffangtasse (13) angeordnet ist, **dadurch gekennzeichnet, dass** der Ablaufschacht (5) in die Auffangtasse (13) hineinragt, die minimale horizontale Querschnittsfläche der Auffangtasse (13) 1,2 bis 4 mal größer ist als die horizontale Querschnittsfläche des Ablaufschachts (5) am Auslass und wobei die Auffangtasse (13) eine umlaufende Wandung (15) mit einem Überlauf (19) aufweist und wobei zur Bildung des Überlaufs Öffnungen in der Wandung (15) der Auffangtasse (13) vorgesehen sind oder die umlaufende Wandung (15) der Auffangtasse (13) zur Bildung des Überlaufs (19) im Bereich des Überlaufs (19) eine geringere Höhe aufweist.

2. Querstromboden gemäß Anspruch 1, **dadurch gekennzeichnet, dass** der Überlauf (19) auf der vom Mantel der Stoffaustauschkolonne (27) abweisenden Seite der Auffangtasse (13) angeordnet ist.

3. Querstromboden gemäß Anspruch 1 oder 2, **dadurch gekennzeichnet, dass** die umlaufende Wandung (15) senkrecht zum Boden der Auffangtasse (13) verläuft.

4. Querstromboden gemäß einem der Ansprüche 1 bis 3, **dadurch gekennzeichnet, dass** der Ablaufschacht (5) eine Querschnittsverengung aufweist, so dass die horizontale Querschnittsfläche des Ablaufschachts (5) am Einlass größer ist als die horizontale Querschnittsfläche am Auslass.

5. Querstromboden gemäß Anspruch 4, **dadurch gekennzeichnet, dass** das Verhältnis der horizontalen Querschnittsfläche am Einlass zur horizontalen Querschnittsfläche am Auslass im Bereich von 1:1 bis 4:1 liegt.

6. Querstromboden gemäß einem der Ansprüche 1 bis 5, **dadurch gekennzeichnet, dass** der Ablaufschacht (5) symmetrisch zu einer vertikal mittig im Ablaufschacht (5) verlaufenden Symmetrieebene ausgebildet ist.

7. Querstromboden gemäß Anspruch 6, **dadurch gekennzeichnet, dass** die parallel zur Symmetrieebene verlaufenden Wandungen (9) des Ablaufschachtes (5) einen Bereich (23) aufweisen, in dem die Wandung in einem Winkel zwischen 10 und 80° geneigt zur Vertikalen verläuft, um die Querschnittsverengung auszubilden.

8. Querstromboden gemäß Anspruch 7, **dadurch gekennzeichnet, dass** die Wandung (9) des Ablaufschachtes (5) oberhalb und unterhalb des zur Vertikalen geneigten Bereichs vertikal verläuft.

9. Querstromboden gemäß einem der Ansprüche 1 bis 8, **dadurch gekennzeichnet, dass** alle Ablaufschächte (5) so positioniert sind, dass der Abstand zwischen der durch den Querstromboden (1) ragenden Wandung (9) des Ablaufschachtes (5) und dem äußeren Rand des Querstrombodens (1) an jeder Stelle mindestens so groß ist, dass mindestens eine Durchtrittsöffnung (3) im Querstromboden (1) ausgebildet sein kann.

10. Stoffaustauschkolonne zur Durchführung eines Stoffaustauschprozesses, umfassend mindesten zwei Querstromböden (1) gemäß einem der Ansprüche 1 bis 9.

11. Stoffaustauschkolonne gemäß Anspruch 10, **dadurch gekennzeichnet, dass** alle Ablaufschächte (5) eines Querstrombodens (1) in der gleichen Hälfte (7) des Querstrombodens (1) angeordnet sind und jeweils zwei übereinanderliegende Querstromböden (1) so angeordnet sind, dass die Ablaufschächte (5) des oberen Querstrombodens (1) oberhalb der Hälfte des unteren Querstrombodens (1) enden, in der keine Ablaufschächte angeordnet sind.

12. Verwendung der Stoffaustauschkolonne gemäß Anspruch 10 oder 11 zur thermischen Trennung eines (Meth)acrylmonomere enthaltenden Gemischs, bei dem eine Flüssigkeit und ein Gasstrom im Gegenstrom durch die Stoffaustauschkolonne (27) geleitet werden und die (Meth)acrylmonomere entweder im Gasstrom oder in der Flüssigkeit enthalten sind.

13. Verwendung der Stoffaustauschkolonne gemäß Anspruch 12, **dadurch gekennzeichnet, dass** das thermische Trennverfahren eine Kondensation ist, wenn die (Meth)acrylmonomere im Gasstrom enthalten sind und eine Rektifikation, wenn die (Meth)acrylmonomere in der Flüssigkeit enthalten sind.

## Claims

1. A crossflow tray for a mass transfer column (27) in which a gas is conducted in countercurrent to a liquid, the crossflow tray (1) having passage orifices (3) for the gas and at least two downcomers (5), the downcomers (5) projecting beyond the top surface of the crossflow tray (1) and a collecting cup (13) being disposed beneath each downcomer (5), wherein the downcomer (5) projects into the collecting cup (13), the minimum horizontal cross-sectional area of the collecting cup (13) being 1.2 to 4 times greater than the horizontal cross-sectional area of the downcomer (5) at the outlet, and where the collecting cup (13) has a circumferential wall (15) having an overflow (19), and where the overflow is formed by provision of orifices in the wall (15) of the collecting cup (13) or the circumferential wall (15) of the collecting cup (13) forms the overflow (19) by having a lower height in the region of the overflow (19).

2. The crossflow tray according to claim 1, wherein the overflow (19) is disposed on the side of the collecting cup (13) facing away from the shell of the mass transfer column (27).

3. The crossflow tray according to claim 1 or 2,
wherein the circumferential wall (15) runs at right angles to the base of the collecting cup (13).

4. The crossflow tray according to any of claims 1 to 3, wherein the downcomer (5) has a cross-sectional constriction, such that the horizontal cross-sectional area of the downcomer (5) at the inlet is greater than the horizontal cross-sectional area at the outlet.

5. The crossflow tray according to claim 4, wherein the ratio of the horizontal cross-sectional area at the inlet to the horizontal cross-sectional area at the outlet is in the range from 1:1 to 4:1.

6. The crossflow tray according to any of claims 1 to 5, wherein the downcomer (5) is formed symmetrically with respect to a plane of symmetry that runs vertically in the middle of the downcomer (5) .

7. The crossflow tray according to claim 6, wherein the walls (9) of the downcomer (5) that run parallel to the plane of symmetry have a region (23) in which the wall runs at an angle of inclination between 10 and 80° with respect to the vertical, in order to form the cross-sectional constriction.

8. The crossflow tray according to claim 7, wherein the wall (9) of the downcomer (5) runs vertically above and below the region inclined with respect to the vertical.

9. The crossflow tray according to any of claims 1 to 8, wherein all the downcomers (5) are positioned such that the distance between the wall (9) of the downcomer (5) that projects through the crossflow tray (1) and the outer edge of the crossflow tray (1) is at least sufficiently great at every point that at least one passage orifice (3) can be formed in the crossflow tray (1).

10. A mass transfer column for conducting a mass transfer operation, comprising at least two crossflow trays (1) according to any of claims 1 to 9.

11. The mass transfer column according to claim 10, wherein all the downcomers (5) of a crossflow tray (1) are disposed in the same half (7) of the crossflow tray (1) and every two superposed crossflow trays (1) are arranged such that the downcomers (5) of the upper crossflow tray (1) end above the half of the lower crossflow tray (1) in which there are no downcomers.

12. The use of the mass transfer column according to claim 10 or 11 for thermal separation of a mixture comprising (meth)acrylic monomers, in which a liquid and a gas stream are passed in countercurrent through the mass transfer column (27) and the (meth)acrylic monomers are present either in the gas stream or in the liquid.

13. The use of the mass transfer column according to claim 12, wherein the thermal separation process is a condensation when the (meth)acrylic monomers are present in the gas stream and a rectification when the (meth)acrylic monomers are present in the liquid.

## Revendications

1. Plateau à courant transversal destiné à une colonne de transfert de matière (27) dans laquelle un gaz est alimenté à contre-courant d'un liquide, le plateau à courant transversal (1) comportant des ouvertures de passage (3) destinées au gaz et au moins deux tubes de retour (5), les tubes de retour (5) faisant saillie du côté supérieur du plateau à courant transversal (1) et une coupelle collectrice (13) étant disposée au-dessous de chaque tube de retour (5), **caractérisé en ce que** le tube de retour (5) fait saillie dans la coupelle collectrice (13), l'aire horizontale minimale en coupe transversale de la coupelle collectrice (13) est 1,2 à 4 fois supérieure à l'aire horizontale en coupe transversale du tube de retour (5) au niveau de la sortie et la coupelle collectrice (13) comportant une paroi périphérique (15) pourvue d'un trop-plein (19) et des ouvertures étant ménagées dans la paroi (15) de la coupelle collectrice (13) pour former le trop-plein ou la paroi périphérique (15) de la coupelle collectrice (13) ayant une hauteur plus petite dans la zone du trop-plein (19) pour former le trop-plein (19).

2. Plateau à courant transversal selon la revendication 1, **caractérisé en ce que** le trop-plein (19) est disposé du côté de la coupelle collectrice (13) qui est opposé à l'enveloppe de la colonne de transfert de matière (27).

3. Plateau à courant transversal selon la revendication 1 ou 2, **caractérisé en ce que** la paroi périphérique (15) s'étend perpendiculairement au fond de la coupelle collectrice (13).

4. Plateau à courant transversal selon l'une des revendications 1 à 3, **caractérisé en ce que** le tube de retour (5) comporte un rétrécissement en coupe transversale de sorte que l'aire horizontale en coupe transversale du tube de retour (5) au niveau de l'entrée est supérieure à l'aire horizontale en coupe transversale au niveau de la sortie.

5. Plateau à courant transversal selon la revendication 4, **caractérisé en ce que** le rapport de l'aire horizontale en coupe transversale au niveau de l'entrée sur l'aire horizontale en coupe transversale au niveau de la sortie est dans la gamme allant de 1:1 à 4:1.

6. Plateau à courant transversal selon l'une des revendications 1 à 5, **caractérisé en ce que** le tube de retour (5) est formé symétriquement à un plan de symétrie s'étendant verticalement au centre du tube de retour (5).

7. Plateau à courant transversal selon la revendication 6, **caractérisé en ce que** les parois (9) du tube de retour (5), qui s'étendent parallèlement au plan de symétrie, comportent une zone (23) dans laquelle la paroi est inclinée d'un angle compris entre 10 et 80° par rapport à la verticale pour former le rétrécissement en coupe transversale.

8. Plateau à courant transversal selon la revendication 7, **caractérisé en ce que** la paroi (9) du tube de retour (5) s'étend verticalement au-dessus et au-dessous de la zone inclinée par rapport à la verticale.

9. Plateau à courant transversal selon l'une des revendications 1 à 8, **caractérisé en ce que** tous les tubes de retour (5) sont positionnés de telle sorte que la distance entre la paroi (9) du tube de retour (5), qui fait saillie à travers le plateau à courant transversal (1), et le bord extérieur du plateau à courant transversal (1) ait en chaque point une dimension telle qu'au moins une ouverture de passage (3) puisse être ménagée dans le plateau à courant transversal (1).

10. Colonne de transfert de matière destinée à mettre en œuvre un procédé de transfert de matière, ladite colonne comprenant au moins deux plateaux à courant transversal (1) selon l'une des revendications 1 à 9.

11. Colonne de transfert de matière selon la revendication 10, **caractérisée en ce que** tous les tubes de retour (5) d'un plateau à courant transversal (1) sont disposés dans la même moitié (7) du plateau à courant transversal (1) et deux plateaux à courant transversal superposés (1) sont disposés de telle sorte que les tubes de retour (5) du plateau à courant transversal supérieur (1) se terminent au-dessus de la moitié du plateau à courant transversal inférieur (1) dans lequel aucun tube de retour n'est disposé.

12. Utilisation de la colonne de transfert de matière selon la revendication 10 ou 11 pour la séparation thermique d'un mélange qui contient des monomères (méth)acryliques et dans lequel un liquide et un flux de gaz sont passés à contre-courant à travers la colonne de transfert de matière (27) et les monomères (méth)acryliques sont contenus dans le flux de gaz ou dans le liquide.

13. Utilisation de la colonne de transfert de matière selon la revendication 12, **caractérisée en ce que** le procédé de séparation thermique est une condensation lorsque les monomères (méth)acryliques sont contenus dans le flux de gaz et une rectification lorsque les monomères (méth)acryliques sont contenus dans le liquide.
